# EUROPEAN PATENT SPECIFICATION

(11) **EP 3 991 669 B1**
(45) Date of publication and mention of the grant of the patent: **18.12.2024**
(21) Application number: 20833409.4
(22) Date of filing: 10.06.2020
(51) Int. Cl.: A61B 17/34, A61B 17/00

(54) **ADSORPTION HEAD AND ADSORPTION DEVICE**
ADSORPTIONSKOPF UND ADSORPTIONSVORRICHTUNG
TÊTE D'ADSORPTION ET DISPOSITIF D'ADSORPTION

(30) Priority: 25.06.2019 CN 201910555241
(43) Date of publication of application: 04.05.2022
(73) Proprietor: Deke Medtech (Hangzhou) Inc., Hangzhou, Zhejiang 310051 (CN)
(72) Inventor: SHAO, Nan, Hangzhou, Zhejiang 310051 (CN); WU, Zhiming, Hangzhou, Zhejiang 310051 (CN); ZI, Zhenjun, Hangzhou, Zhejiang 310051 (CN); HU, Jingjing, Hangzhou, Zhejiang 310051 (CN); XU, Mingyuan, Hangzhou, Zhejiang 310051 (CN)
(74) Representative: Novagraaf Group
(86) International application number: PCT/CN2020/095440
(87) International publication number: WO 2020/259292

(56) References cited:
- EP-A1- 1 328 207
- CN-A- 103 338 717
- CN-A- 104 546 078
- CN-A- 104 546 078
- CN-A- 106 236 212
- CN-A- 106 725 765
- CN-A- 106 725 765
- CN-A- 108 245 228
- CN-U- 204 446 041
- CN-U- 204 500 896
- CN-U- 209 529 200
- US-A- 5 984 864
- US-A- 6 032 672
- US-A1- 2014 276 986

## Description

### TECHNICAL FIELD

The present disclosure relates to the field of medical technology, and particularly to a puncture needle assembly and a puncture adsorption mechanism thereof.

### BACKGROUND

Among many surgeries requiring precise operations, how to enable a medical instrument to be in a stable state relative to a living body has always been a medical difficulty. For example, a cardiac surgery is performed on a beating heart all the time since the heart is unable to suspend during the surgery, which greatly increases the surgical difficulty. CN104546078A discloses a clinical puncture guidance positioning and fixing device, which includes an annular suction type suction cup, a supporting disk, a rotating disk and an arc-shaped arm.

In a specific operation, a doctor is difficult to achieve cardiac puncture and infusion of compounds after puncture since the heart is beating. Because the heart is beating, the puncture needle is prone to slip during the puncture and further deviate from a target point. How to make the operation of the puncture needle, an endoscope and other operation instruments on the heart more stable has become a difficulty.

### SUMMARY

In view of this, it is necessary to provide a puncture adsorption mechanism for the above technical problems, which may enable an adsorption device to be stably connected to a viable tissue and facilitate a stable operation of a surgical instrument on a surface of the viable tissue. Claims 1 and 11 defines the invention and dependent claims disclose embodiments. No surgical methods are claimed.

In order to achieve the above objective, the present disclosure adopts the following technical solution. An adsorption head includes a body, wherein the body is a hollow housing, one side of the housing to which an adsorbed object is adsorbed is provided with an opening; the body comprises at least one adsorption chamber therein and at least one operation chamber, wherein the operation chamber and the adsorption chamber are spaced from each other and each have an opening orientated to the adsorbed object; and the body has at least one adsorption passage and at least one operation passage thereon, wherein one end of the adsorption passage is communicated with the adsorption chamber, and one end of the operation passage is communicated with the operation chamber. The at least one operation chamber is defined within the body of the housing. The body has a first adsorption chamber and a second adsorption chamber therein, and the first adsorption chamber is communicated with the second adsorption chamber through a communication structure which includes a connection tube arranged on a side wall of the operation chamber.

The above solution has the following advantages that the adsorption head is adsorbed to a surface of a living body, and further the adsorption head can be adsorbed to the surface of a viable tissue; an endoscope or a puncture needle is extended into the operation chamber so that the endoscope or the puncture needle is stable relative to the operation chamber; then the living body is subjected to a surgical operation; and because the adsorption head 5 is adsorbed to the surface of the living body and is static relative to the surface of the living body, the surgical instrument is kept stable relative to the surface of the living body and has a relatively high controllability for the operation on the living body, which brings the convenience for smoothly performing the surgery.

In a feasible solution, the first adsorption chamber and/or the second adsorption chamber are/is communicated with the adsorption passage.

By providing the two adsorption chambers, the adsorption head can be more firmly adsorbed to the surface of the living body, which brings the convenience for performing the surgery. Moreover, the first adsorption chamber and the second adsorption chamber are communicated with each other, such that the first adsorption chamber and the second adsorption chamber have the same air pressure therein and have the same adsorption force during adsorption.

The connection tube communicating the first communicating chamber with the second adsorption chamber is detachable and convenient to replace.

In a feasible solution, the communication structure further includes an accommodation passage defined in the body, and the connection tube is disposed within the accommodation passage and communicates the first adsorption chamber with the second adsorption chamber. By disposing the accommodation passage within the body and disposing the connection tube within the accommodation passage, the whole body is relatively flat, and is conveniently extended into the living body for performing an adsorption operation.

In a feasible solution, the connection tube has a front end and a rear end, wherein the front end and the rear end of the connection tube are provided with an opening, the opening in the rear end is communicated with the first adsorption chamber, and the opening in the front end is communicated with the second adsorption chamber.

In a feasible solution, the front end is provided with a first extension section extending along an axial direction of the connection tube. With this arrangement, a certain distance exists between the connection tube and the body, which prevents air circulation within the connection tube from being blocked.

In a feasible solution, the first extension section is of an arc-shaped structure. The arc-shaped structure has better support without blocking the air circulation within the connection tube, and is convenient to manufacture.

In a feasible solution, the rear end is provided with a second extension section extending along the axial direction of the connection tube.

With this arrangement, a certain distance exists between the connection tube and the body, which prevents the air circulation within the connection tube from being blocked.

In a feasible solution, the second extension section is of an arc-shaped structure. The arc-shaped structure has good support without blocking the air circulation within the connection tube, and is convenient to manufacture.

As stated above, the connection tube is disposed on a side wall of the operation chamber. Therefore, the operation chamber will not be blocked, which brings the convenience for the use of a surgical instrument.

In a feasible solution, two said connection tubes are disposed between the first adsorption chamber and the second adsorption chamber, and respectively positioned on two opposite side walls of the operation chamber. Accordingly, the air circulation between the first adsorption chamber and the second adsorption chamber is relatively great, and no space within the operation chamber will be occupied by the connection tube. As a result, obstructions to the use of the surgical instrument are avoided.

In a feasible solution, the first adsorption chamber and the second adsorption chamber are respectively communicated with the two adsorption passages. With this arrangement, the first adsorption chamber and the second adsorption chamber may be connected with different suction devices respectively, has and thus better adjustment flexibility is achieved.

In a feasible solution, a side surface of one side of the body which is opposite to the opening is an arc-shaped surface, which brings the convenience for an adsorption head to extend into the living body for adsorption.

In a feasible solution, a first operation passage and a second operation passage are defined in the body, and both communicated with the operation chamber. The first operation passage and the second operation passage are respectively extended into different surgical instruments, such that the surgical instruments will not interfere with each other.

In a feasible solution, a cross-sectional area of the first operation passage is greater than that of the second operation passage, which brings the convenience for a surgical instrument with a larger diameter into the first operation passage.

In a feasible solution, the second operation passage is arranged obliquely relative to a surface of an adsorbed object. Such an oblique arrangement brings the convenience for the surgical instrument to extend into the living body, for example, the puncture needle to puncture the living body.

In comparison with the prior art, the adsorption head is internally provided with the adsorption chamber and the operation chamber. When the adsorption head is adsorbed to the surface of the viable tissue, the surgical instrument positioned within the operation chamber may be static relative to the surface of the viable tissue, thereby improving the operation stability and the success rate of the surgical operation, and reducing the probability of causing the secondary damage. Meanwhile, by providing multiple adsorption chambers, the adsorption head can be more stably adsorbed to the surface of the living body, which brings the convenience for the surgical operation.

The prevent disclosure further provides an adsorption device, which includes a suction device and any one of the aforementioned adsorption heads, wherein the suction device is connected with the adsorption passage, and the adsorption device further includes a support tube and extension sections of the adsorption passage and the operation passage are disposed within the support tube. The suction device is connected to the adsorption chamber through the adsorption passage. The adsorption head can be adsorbed to the surface of the viable tissue by means of the suction of the suction device.

In a feasible solution, one end of the support tube is connected to the adsorption head and another end thereof is provided with an opening. The surgical instrument is extended into the support tube and further into the operation chamber for performing a surgical operation.

In a feasible solution, the support tube has a flexible connection section. By providing the flexible connection section, the support tube may be bent so as to conveniently extend into the living body. Moreover, when the adsorption head is displaced with the living body, the flexible connection section may play a role of connection and maintain the stability of other parts of the support tube.

In a feasible solution, the support tube includes a first support tube, a flexible connection section and a second support tube connected in sequence, and the first support tube is connected to the adsorption head.

In comparison with the prior art, the adsorption device can be extended into the living body for performing the surgical operation, and provided with the adsorption head. The adsorption head is adsorbed to the surface of the viable tissue by means of the suction of the suction device, such that during the surgical operation, better stability between the surgical instrument and the viable tissue is achieved, the convenience is brought for the operation and the success rate of the surgery is improved.

### BRIEF DESCRIPTION OF THE DRAWINGS

FIG. 1 is a perspective view according to the present disclosure;
**[0031]**FIG. 2 is a right side view according to the present disclosure;

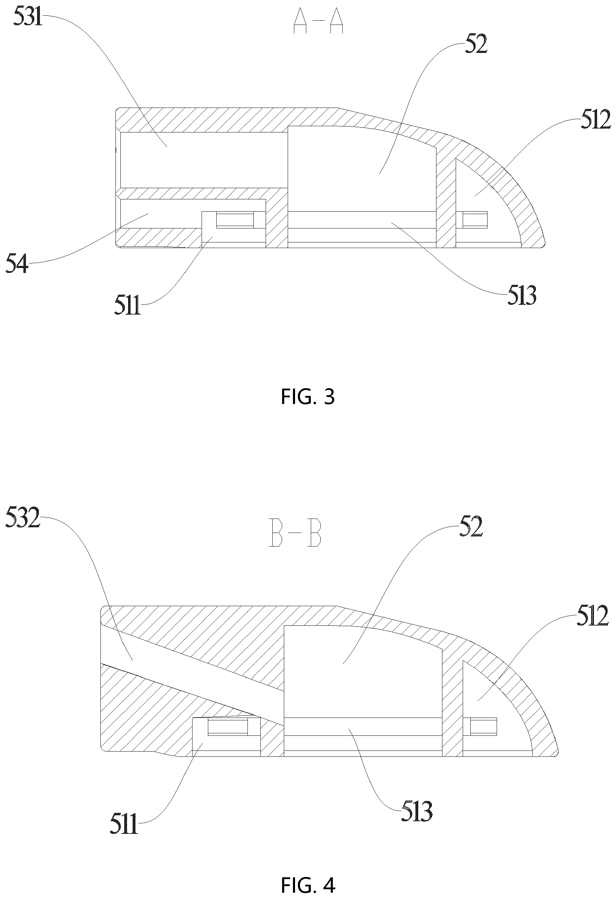

FIG. 3 is a cross-sectional view in a direction A-A of FIG. 2 according to the present disclosure;
FIG. 4 is a cross-sectional view in a direction B-B of FIG. 2 according to the present disclosure;
FIG. 5 is a perspective view showing a connection tube according to the present disclosure; and
FIG. 6 is a perspective view showing an adsorption device according to the present disclosure.

In the figures, 5: adsorption head; 50: body; 51: adsorption chamber; 511: first adsorption chamber; 512: second adsorption chamber; 513: connection tube; 5131: first extension section; 5132: second extension section; 52: operation chamber; 53: operation passage; 531: first operation passage; 532: second operation passage; 54: adsorption passage; 55: support tube; 551: first support tube; 552: flexible connection section; and 553: second support tube.

### DESCRIPTION OF THE EMBODIMENTS

Technical solutions in embodiments of the present disclosure will be clearly and completely described below in conjunction with accompanying drawings in the embodiments of the present disclosure. Obviously, the described embodiments are only some, but not all of the embodiments of the present disclosure. Based on the embodiments of the present disclosure, all other embodiments obtained by those ordinarily skilled in the art without any creative labor shall fall within a protective scope of the present disclosure.

It should be noted that when a component is referred to as being "installed on" another component, it may be directly on another component or an intervening component may exist therebetween as well. When a component is considered to be "installed on "another component, it may be directly installed on another component or an intervening component may exist therebetween simultaneously. When a component is considered to be "fixed on" another component, it may be directly fixed on another component or an intervening component may exist therebetween simultaneously.

Unless otherwise defined, all technical and scientific terms used herein have the same meaning as commonly understood by those skilled in the technical field of the present disclosure. The terms used in the description of the present disclosure herein are only for the purpose of describing specific embodiments, and not intended to limit the present disclosure. The term "or/and" as used herein includes any and all combinations of one or more related listed items.

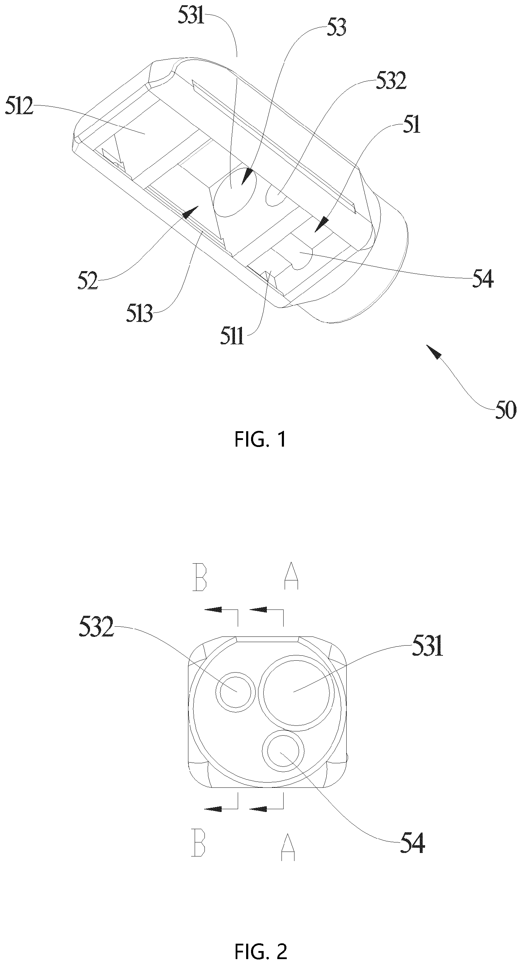

As shown in FIG. 1, the present disclosure provides an adsorption head 5, including a body 50, wherein the body 50 is a hollow housing, the housing is provided with an opening, and the opening is oriented towards an adsorbed object. The body 50 comprises at least one adsorption chamber 51 and at least one operation chamber 52 therein, wherein the operation chamber 52 and the adsorption chamber 51 are spaced from each other and each have an opening orientated to the adsorbed object. The body 50 has at least one adsorption passage 54 and at least one operation passage 53 thereon, wherein one end of the adsorption passage 54 is communicated with the adsorption chamber 51, and one end of the operation passage 53 is communicated with the operation chamber 52.

It should be noted that the adsorption chamber 51 can be adsorbed to a surface of a living body by connecting a suction device, so that the adsorption head 5 can be adsorbed to the surface of the viable tissue. An endoscope or a puncture needle is extended into the operation chamber 52 through the operation passage 53, so that the endoscope or the puncture needle is stable relative to the operation chamber 52. When a surgical operation is performed on the living body, since the adsorption head 50 is adsorbed to the surface of the living body and moves synchronously with the surface of the living body, while the operation chamber 52 and the adsorption chamber 51 are fixed relative to each other, an surgical instrument can be kept stable relative to the surface of the living body, and further has a relatively high controllability for the operation on the living body, which brings the convenience for smoothly performing the surgery.

Preferably, the number of the adsorption chambers 51 is two, and the two adsorption chambers 51 are the first adsorption chamber 511 and the second adsorption chamber 512 respectively. The first adsorption chamber 511 and the second adsorption chamber 512 are communicated with each other. The first adsorption chamber 511 and/or the second adsorption chamber 512 are/is communicated with the adsorption passage 54 respectively. Certainly, in other embodiments, the number of the adsorption chambers 51 may be any other number, for example, 3 or 4. The specific number of the adsorption chambers may be set according to actual requirements.

It should be understood that by providing the two adsorption chambers 51, the adsorption head 50 may be more firmly adsorbed to the surface of the living body, and further brings the convenience for performing the surgery; secondly, the first adsorption chamber 511 and the second adsorption chamber 512 are communicated with each other, so that air pressures within the first adsorption chamber 511 and the second adsorption chamber 512 are the same. The adsorption chamber 51 has the same pressure during adsorption, such that the adsorption force of the adsorption chamber 51 to the surface of the living body is conveniently controlled and the surface of the living body is prevented from being damaged by excessive adsorption strength.

Preferably, the first adsorption chamber 511 and the second adsorption chamber 512 are communicated with each other through a communication structure.

More preferably, the communication structure may be a passage, a through hole, or the like.

Just for illustration, the communication structure is a communication passage defined in the body 50 which does not form part of the invention. Accordingly, the first adsorption chamber 511 and the second adsorption chamber 512 are communicated through the communication passage, such that a certain distance exists between the first adsorption chamber 511 and the second adsorption chamber 512, which makes arrangement positions of the first adsorption chamber 511 and the second adsorption chamber 52 be more flexible and facilitates the adsorption of the adsorption head 5.

In another embodiment, the communication structure is a connection tube 513, wherein the connection tube 513 communicates the first adsorption chamber 511 with the second adsorption chamber 512, and the connection tube 513 is an independent part and is detachable. It should be understood that the connection tube 513 connects the first adsorption chamber 511 with the second adsorption chamber 512, which is simple in structure and is more convenient for replacement, maintenance, processing and installation.

Preferably, in this embodiment, the body 50 has an accommodation passage (not illustrated), and the connection tube 513 is disposed within the accommodation passage and communicates the first adsorption chamber 511 with the second adsorption chamber 512. It should be understood that by disposing the accommodation passage within the body 50 and disposing the connection tube 513 within the accommodation passage, the whole body 50 is relatively flat, and is conveniently extended into the living body for performing an adsorption operation.

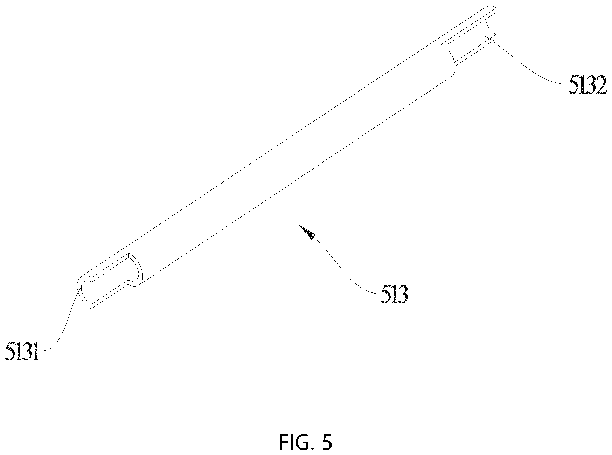

As shown in FIG. 5, the connection tube 513 has a front end and a rear end arranged opposite to each other. The front end and the rear end of the connection tube 513 are provided with an opening, and the opening in the rear end is communicated with the first adsorption chamber 511 and the opening in the front end is communicated with the second adsorption chamber 512.

Further, the front end is provided with a first extension section 5131 extending along an axial direction of the connection tube 513.

Preferably, the first extension section 5131 is of an arc-shaped structure.

Further, the rear end is provided with a second extension section 5132 extending along the axial direction of the connection tube 513.

Preferably, the second extension section 5132 is of an arc-shaped structure.

It should be understood that by providing the first extension section 5131 and the second extension section 5132, the opening of the connection tube 513 will not be blocked by other structures within the extension section, such that when the connection tube 513 communicates the two adsorption chambers 51, air circulates more smoothly. Next, the first extension section 5131 and the second extension section 5132 are arranged to be of an arc-shaped structure, and are an extension of a tube wall of the connection tube 513 on a side away from the adsorption chamber 51, such that the extension section will not block air circulation between the connection tube 513 and the adsorption chamber 51. Moreover, the extension section is convenient to manufacture.

Preferably, the connection tube 513 is disposed on a side wall of the operation chamber 52, such that the surgical instrument will not be blocked by the connection tube 513 when being extended into the operation chamber 52 for performing a surgical operation, which facilitates the use of the surgical instrument.

Preferably, two said connection tubes 513 are provided between the first adsorption chamber 511 and the second adsorption chamber 512, and respectively positioned on two opposite side walls of the operation chamber 52. It should be understood that by providing the two said connection tubes 513, the air circulation between the first adsorption chamber 511 and the second adsorption chamber 512 is relatively great, and the adsorption effect is better. Certainly, the number of the connection tubes 513 may be selected to be more, for example, 3 and 4.

As another embodiment, the body 50 has a first adsorption chamber 511 and a second adsorption chamber 512 therein. Moreover, the body 50 is internally provided with two adsorption passages 54. The first adsorption chamber 511 and the second adsorption chamber 512 are communicated with one of the adsorption passages 54 respectively. With this arrangement, the first adsorption chamber 511 and the second adsorption chamber 512 may be respectively connected with different suction devices, thereby having a better flexibility in adjusting an adsorption mode.

Preferably, a side surface of one side of the body 50 which is opposite to the opening is an arc-shaped surface. It should be understood that one side of the body 50 is set to be of an arc-shaped structure, which may bring the convenience for the adsorption head 5 to extend into the living body.

Preferably, the body 50 has a first operation passage 531 and a second operation passage 532 thereon, wherein the first operation passage 531 and the second operation passage 532 are communicated with the operation chamber 52. The first operation passage 531 and the second operation passage 532 are respectively configured to extend into different surgical instruments, such that the surgical instruments will not interfere with each other during use. Certainly, in other embodiments, more operation passages 53 may be provided on the body 50, and the number of the operation passages 53 is not limited, which may be set according to actual requirements.

Preferably, a cross-sectional area of the first operation passage 531 is greater than that of the second operation passage 532. It should be understood that setting the cross-sectional area of the first operation passage 531 and the cross-sectional area of the second operation passage 532 differently may facilitate the extension of a surgical instrument with a larger diameter into the first operation passage 531.

Preferably, the second operation passage 532 is arranged obliquely relative to the surface of an adsorbed object.

It should be noted that such an oblique arrangement of the operation passage 53 may bring the convenience for the surgical instrument to extend into the living body, for example, the puncture needle to puncture the living body.

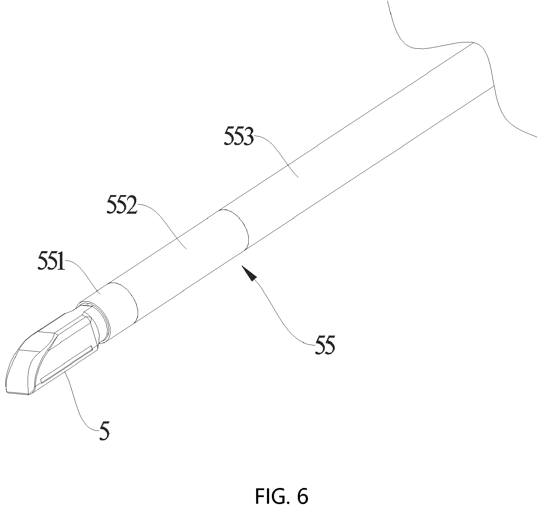

As shown in FIG. 6, the present disclosure further provides an adsorption device, including a suction device and an adsorption head 5, wherein the suction device is connected with the adsorption passage 54, and a specific structure of the adsorption head may refer to the adsorption head 5 described above in the detailed description. The suction device is connected to the adsorption chamber 51 through the adsorption passage 54, and the adsorption head 5 may be adsorbed to a surface of a viable tissue by means of the suction of the suction device.

It should be noted that in one of the embodiments, the suction device may be one, and is communicated with the adsorption chamber 51 through the adsorption passage 54; and in another embodiment, the number of the suction devices may be more, and the suction devices may be respectively connected with different adsorption chambers 51.

Preferably, the adsorption device further includes a support tube (not illustrated), wherein one end of the support tube is connected to the adsorption head 5 and the other end of the support tube is provided with an opening, and the surgical instrument is extended into the support tube and further into the operation chamber 52 for performing a surgical operation.

It should be understood that by providing the support tube, the adsorption head 5 may be better controlled, and extension sections of the adsorption passage 54 and the operation passage 53 may be disposed within the support tube, which brings the convenience for performing the surgical operation.

Preferably, the support tube has a flexible connection section 552. By providing the flexible connection section 552, the support tube may be bent so as to conveniently extend into the living body. Moreover, when the adsorption head is displaced with the living body, the flexible connection section may play a role of connection and maintain the stability of other parts of the support tube.

In an embodiment, the support tube includes a first support tube 551, a flexible connection section 552 and a second support tube 553 connected in sequence, wherein the first support tube 551 is connected to the adsorption head 5.

In another embodiment, the supporting tube includes a flexible connection section 552 and a second support tube 553, wherein the flexible connection section 552 is directly connected with the adsorption head.

It should be understood that the flexible connection section 552 is provided in various fashions to allow the support tube to be bent, for example, a plurality of flexible connection sections 552 are provided, wherein the plurality of flexible connection sections 552 may be arranged at intervals, the whole support tube is of a flexible structure, or the like.

In comparison with the prior art, the adsorption device can be extended into the living body for performing the surgical operation, and provided with the adsorption head 5. By means of the suction of the suction device, the adsorption head 5 is adsorbed to the surface of the viable tissue, such that when the surgical operation is performed, a better stability is maintained between the surgical instrument and the viable tissue. Furthermore, the surgery is facilitated, and the success rate of the surgery is increased.

Technical features of the embodiments mentioned above may be combined arbitrarily. In order to make the description concise, all possible combinations of various technical features in the embodiments mentioned above are not described. However, the combinations of these technical features should be considered as the scope recorded in the specification provided that no contradiction exists.

The embodiments mentioned above only describe several implementations of the present disclosure more particularly in detail, but should not be understood as limiting the scope of the present disclosure. It should be noted that for those ordinarily skilled in the art, several modifications and improvements may be made without departing from the concept of the present disclosure, and all fall within a protective scope of the present disclosure. The scope of the invention is defined by appended claims.

## Claims

1. An adsorption head (5), comprising:
a body (50), wherein the body (50) is a hollow housing, one side of the housing to which an adsorbed object is adsorbed is provided with an opening; the body (50) comprises at least one adsorption chamber (51; 511; 512) therein and at least one operation chamber (52), wherein the operation chamber (52) and the adsorption chamber (51; 511; 512) are spaced from each other and each have an opening orientated to the adsorbed object; and the body (50) has at least one adsorption passage (54) and at least one operation passage (53; 531; 532) thereon, wherein one end of the adsorption passage (54) is communicated with the adsorption chamber (51; 511; 512), and one end of the operation passage (53; 531; 532) is communicated with the operation chamber (52), **characterized in that**, the at least one operation chamber (52) is defined within the body (50) of the housing, the body (50) has a first adsorption chamber (511) and a second adsorption chamber (512) therein, and the first adsorption chamber (511) is communicated with the second adsorption chamber (512) through a communication structure which comprises a connection tube (513) arranged on a side wall of the operation chamber (52).

2. The adsorption head (5) according to claim 1, wherein the adsorption head (5) further has additional feature a), b), c) or d):
a) the first adsorption chamber (511) and/or the second adsorption chamber (512) are/is communicated with the adsorption passage (54);
b) the first adsorption chamber (511) and the second adsorption chamber (512) are respectively communicated with the two adsorption passages (54);
c) a side surface of one side of the body (50) which is opposite to the opening is an arc-shaped surface;
d) a first operation passage (531) and a second operation passage (532) are defined in the body (50), and both communicated with the operation chamber (52).

3. The adsorption head (5) according to claim 2, wherein when the adsorption head (5) has the feature a), the adsorption head (5) further has additional feature f):
f) the communication structure further comprises an accommodation passage defined in the body (50), and the connection tube (513) is disposed within the accommodation passage and communicates the first adsorption chamber (511) with the second adsorption chamber (512).

4. The adsorption head (5) according to claim 3, wherein the adsorption head (5) further has additional feature g):
g) the connection tube (513) has a front end and a rear end, wherein both the front end and the rear end of the connection tube (513) are provided with an opening, the opening in the rear end is communicated with the first adsorption chamber (511), and the opening in the front end is communicated with the second adsorption chamber (512).

5. The adsorption head (5) according to claim 4, wherein the front end is provided with a first extension section (5131) extending along an axial direction of the connection tube (513).

6. The adsorption head (5) according to claim 5, wherein the first extension section (5131) is of an arc-shaped structure.

7. The adsorption head (5) according to claim 4, wherein the rear end is provided with a second extension section (5132) extending along the axial direction of the connection tube (513).

8. The adsorption head (5) according to claim 7, wherein the second extension section (5132) is of an arc-shaped structure.

9. The adsorption head (5) according to claim 1, wherein two said connection tubes (513) are provided between the first adsorption chamber (511) and the second adsorption chamber (512), and respectively positioned on two opposite side walls of the operation chamber (52).

10. The adsorption head (5) according to claim 2, wherein when the adsorption head (5) has the feature d), the adsorption head (5) further has at least one of additional features i) and m):
i) a cross-sectional area of the first operation passage (531) is greater than that of the second operation passage (532);
m) the second operation passage (532) is arranged obliquely relative to a surface of an adsorbed obj ect.

11. An adsorption device, **characterized in that**, the adsorption device comprising a suction device and the adsorption head (5) according to any one of claims 1 to 10, wherein the suction device is connected with the adsorption passage (54), and the adsorption device further comprises a support tube (55) and extension sections of the adsorption passage (54) and the operation passage (53; 531; 532) are disposed within the support tube (55).

12. The adsorption device according to claim 11, wherein the adsorption device further comprises additional feature p), or features p) and q):
p) wherein one end of the support tube (55) is connected to the adsorption head (5) and the other end of the support tube (55) is provided with an opening;
q) the support tube (55) has a flexible connection section (552).

13. The adsorption device according to claim 12, wherein the flexible connection section (552) of the support tube (55) is directly connected with the adsorption head (5).

14. The adsorption device according to claim 11, wherein the support tube (55) comprises a first support tube (551), a flexible connection section (552) and a second support tube (553) connected in sequence, and the first support tube (551) is connected to the adsorption head (5).

## Patentansprüche

1. Adsorptionskopf (5), umfassend:
einen Körper (50), wobei der Körper (50) ein hohles Gehäuse ist, wobei eine Seite des Gehäuses, an der ein adsorbiertes Objekt adsorbiert wird, mit einer Öffnung versehen ist; wobei der Körper (50) darin mindestens eine Adsorptionskammer (51; 511; 512) und mindestens eine Betriebskammer (52) umfasst, wobei die Betriebskammer (52) und die Adsorptionskammer (51; 511; 512) voneinander beabstandet sind und jeweils eine Öffnung aufweisen, die auf das adsorbierte Objekt ausgerichtet ist; und wobei der Körper (50) mindestens einen Adsorptionsdurchgang (54) und mindestens einen Betriebsdurchgang (53; 531; 532) daran aufweist, wobei ein Ende des Adsorptionsdurchgangs (54) mit der Adsorptionskammer (51; 511; 512) verbunden ist und ein Ende des Betriebsdurchgangs (53; 531; 532) mit der Betriebskammer (52) verbunden ist, **dadurch gekennzeichnet, dass** die mindestens eine Betriebskammer (52) innerhalb des Körpers (50) des Gehäuses definiert ist, der Körper (50) eine erste Adsorptionskammer (511) und eine zweite Adsorptionskammer (512) darin aufweist und die erste Adsorptionskammer (511) mit der zweiten Adsorptionskammer (512) durch eine Kommunikationsstruktur verbunden ist, die ein Verbindungsrohr (513) umfasst, das an einer Seitenwand der Betriebskammer (52) angeordnet ist.

2. Adsorptionskopf (5) nach Anspruch 1, wobei der Adsorptionskopf (5) ferner ein zusätzliches Merkmal a), b), c) oder d) aufweist:
a) die erste Adsorptionskammer (511) und/oder die zweite Adsorptionskammer (512) ist/sind mit dem Adsorptionsdurchgang (54) verbunden;
b) die erste Adsorptionskammer (511) und die zweite Adsorptionskammer (512) sind jeweils mit den zwei Adsorptionsdurchgängen (54) verbunden;
c) eine Seitenoberfläche einer Seite des Körpers (50), die der Öffnung gegenüberliegt, ist eine bogenförmige Oberfläche;
d) ein erster Betriebsdurchgang (531) und ein zweiter Betriebsdurchgang (532) sind in dem Körper (50) definiert und beide sind mit der Betriebskammer (52) verbunden.

3. Adsorptionskopf (5) nach Anspruch 2, wobei, wenn der Adsorptionskopf (5) das Merkmal a) aufweist, der Adsorptionskopf (5) ferner ein zusätzliches Merkmal f) aufweist:
f) die Kommunikationsstruktur umfasst ferner einen in dem Körper (50) definierten Aufnahmedurchgang, und das Verbindungsrohr (513) ist innerhalb des Aufnahmedurchgangs angeordnet und verbindet die erste Adsorptionskammer (511) mit der zweiten Adsorptionskammer (512).

4. Adsorptionskopf (5) nach Anspruch 3, wobei der Adsorptionskopf (5) ferner ein zusätzliches Merkmal g) aufweist:
g) das Verbindungsrohr (513) weist ein vorderes Ende und ein hinteres Ende auf, wobei sowohl das vordere Ende als auch das hintere Ende des Verbindungsrohrs (513) mit einer Öffnung versehen sind, wobei die Öffnung im hinteren Ende mit der ersten Adsorptionskammer (511) verbunden ist und die Öffnung im vorderen Ende mit der zweiten Adsorptionskammer (512) verbunden ist.

5. Adsorptionskopf (5) nach Anspruch 4, wobei das vordere Ende mit einem ersten Verlängerungsabschnitt (5131) versehen ist, der sich entlang einer axialen Richtung des Verbindungsrohrs (513) erstreckt.

6. Adsorptionskopf (5) nach Anspruch 5, wobei der erste Verlängerungsabschnitt (5131) eine bogenförmige Struktur aufweist.

7. Adsorptionskopf (5) nach Anspruch 4, wobei das hintere Ende mit einem zweiten Verlängerungsabschnitt (5132) versehen ist, der sich entlang der axialen Richtung des Verbindungsrohrs (513) erstreckt.

8. Adsorptionskopf (5) nach Anspruch 7, wobei der zweite Verlängerungsabschnitt (5132) eine bogenförmige Struktur aufweist.

9. Adsorptionskopf (5) nach Anspruch 1, wobei zwei der Verbindungsrohre (513) zwischen der ersten Adsorptionskammer (511) und der zweiten Adsorptionskammer (512) vorgesehen sind und jeweils an zwei gegenüberliegenden Seitenwänden der Betriebskammer (52) positioniert sind.

10. Adsorptionskopf (5) nach Anspruch 2, wobei, wenn der Adsorptionskopf (5) das Merkmal d) aufweist, der Adsorptionskopf (5) ferner eines von zusätzlichen Merkmalen i) und m) aufweist:
i) eine Querschnittsfläche des ersten Betriebsdurchgangs (531) ist größer als die des zweiten Betriebsdurchgangs (532);
m) der zweite Betriebsdurchgang (532) ist schräg zu einer Oberfläche eines adsorbierten Objekts angeordnet.

11. Adsorptionsvorrichtung, **dadurch gekennzeichnet, dass** die Adsorptionsvorrichtung eine Saugvorrichtung und den Adsorptionskopf (5) nach einem der Ansprüche 1 bis 10 umfasst, wobei die Saugvorrichtung mit dem Adsorptionsdurchgang (54) verbunden ist und die Adsorptionsvorrichtung ferner ein Stützrohr (55) umfasst und Verlängerungsabschnitte des Adsorptionsdurchgangs (54) und des Betriebsdurchgangs (53; 531; 532) innerhalb des Stützrohrs (55) angeordnet sind.

12. Adsorptionsvorrichtung nach Anspruch 11, wobei die Adsorptionsvorrichtung ferner ein zusätzliches Merkmal p) oder zusätzliche Merkmale p) und q) umfasst:
p) wobei ein Ende des Stützrohrs (55) mit dem Adsorptionskopf (5) verbunden ist und das andere Ende des Stützrohrs (55) mit einer Öffnung versehen ist;
q) das Stützrohr (55) weist einen flexiblen Verbindungsabschnitt (552) auf.

13. Adsorptionsvorrichtung nach Anspruch 12, wobei der flexible Verbindungsabschnitt (552) des Stützrohrs (55) direkt mit dem Adsorptionskopf (5) verbunden ist.

14. Adsorptionsvorrichtung nach Anspruch 11, wobei das Stützrohr (55) ein erstes Stützrohr (551), einen flexiblen Verbindungsabschnitt (552) und ein zweites Stützrohr (553) umfasst, die in Abfolge verbunden sind, und das erste Stützrohr (551) mit dem Adsorptionskopf (5) verbunden ist.

## Revendications

1. Tête d'adsorption (5), comprenant :
un corps (50), dans laquelle le corps (50) est un boîtier creux, un côté du boîtier auquel un objet adsorbé est adsorbé étant pourvu d'une ouverture ; le corps (50) comprend au moins une chambre d'adsorption (51 ; 511 ; 512) et au moins une chambre de travail (52), dans laquelle la chambre de travail (52) et la chambre d'adsorption (51 ; 511 ; 512) sont espacées les unes des autres et ont chacune une ouverture orientée vers l'objet adsorbé ; et le corps (50) a au moins un passage d'adsorption (54) et au moins un passage de fonctionnement (53 ; 531 ; 532), dans laquelle une extrémité du passage d'adsorption (54) est en communication avec la chambre d'adsorption (51 ; 511 ; 512), et une extrémité du passage de fonctionnement (53 ; 531 ; 532) est en communication avec la chambre de travail (52), **caractérisée en ce que** l'au moins une chambre de travail (52) est définie à l'intérieur du corps (50) du boîtier, le corps (50) a une première chambre d'adsorption (511) et une seconde chambre d'adsorption (512), et la première chambre d'adsorption (511) est en communication avec la seconde chambre d'adsorption (512) par une structure de communication qui comprend un tube de liaison (513) agencé sur une paroi latérale de la chambre de travail (52).

2. Tête d'adsorption (5) selon la revendication 1, dans laquelle la tête d'adsorption (5) présente en outre une caractéristique a), b), c) ou d) supplémentaire :
a) la première chambre d'adsorption (511) et/ou la seconde chambre d'adsorption (512) est/sont en communication avec le passage d'adsorption (54) ;
b) la première chambre d'adsorption (511) et la seconde chambre d'adsorption (512) sont respectivement en communication avec les deux passages d'adsorption (54) ;
c) la surface latérale d'un côté du corps (50) opposé à l'ouverture est une surface en forme d'arc ;
d) un premier passage de fonctionnement (531) et un second passage de fonctionnement (532) sont définis dans le corps (50) et sont tous deux en communication avec la chambre de travail (52).

3. Tête d'adsorption (5) selon la revendication 2, dans laquelle, lorsque la tête d'adsorption (5) présente la caractéristique a), la tête d'adsorption (5) présente en outre la caractéristique f) supplémentaire :
f) la structure de communication comprend en outre un passage de logement défini dans le corps (50), et le tube de liaison (513) est disposé dans le passage de logement et fait communiquer la première chambre d'adsorption (511) avec la seconde chambre d'adsorption (512).

4. Tête d'adsorption (5) selon la revendication 3, dans laquelle la tête d'adsorption (5) présente en outre une caractéristique g) supplémentaire :
g) le tube de liaison (513) a une extrémité avant et une extrémité arrière, dans laquelle l'extrémité avant et l'extrémité arrière du tube de liaison (513) sont toutes deux pourvues d'une ouverture, l'ouverture dans l'extrémité arrière est en communication avec la première chambre d'adsorption (511) et l'ouverture dans l'extrémité avant est en communication avec la seconde chambre d'adsorption (512).

5. Tête d'adsorption (5) selon la revendication 4, dans laquelle l'extrémité avant est pourvue d'une première section d'extension (5131) s'étendant le long d'une direction axiale du tube de liaison (513).

6. Tête d'adsorption (5) selon la revendication 5, dans laquelle la première section d'extension (5131) a une structure en forme d'arc.

7. Tête d'adsorption (5) selon la revendication 4, dans laquelle l'extrémité arrière est pourvue d'une seconde section d'extension (5132) s'étendant le long de la direction axiale du tube de liaison (513).

8. Tête d'adsorption (5) selon la revendication 7, dans laquelle la seconde section d'extension (5132) a une structure en forme d'arc.

9. Tête d'adsorption (5) selon la revendication 1, dans laquelle lesdits deux tubes de liaison (513) sont prévus entre la première chambre d'adsorption (511) et la seconde chambre d'adsorption (512), et positionnés respectivement sur deux parois latérales opposées de la chambre de travail (52).

10. Tête d'adsorption (5) selon la revendication 2, dans laquelle, lorsque la tête d'adsorption (5) présente la caractéristique d), la tête d'adsorption (5) présente en outre au moins l'une parmi des caractéristiques i) et m) supplémentaires :
i) une section transversale du premier passage de fonctionnement (531) est supérieure à celle du second passage de fonctionnement (532) ;
m) le second passage de fonctionnement (532) est agencé obliquement par rapport à la surface d'un objet adsorbé.

11. Dispositif d'adsorption, **caractérisé en ce que** le dispositif d'adsorption comprend un dispositif d'aspiration et la tête d'adsorption (5) selon l'une quelconque des revendications 1 à 10, dans lequel le dispositif d'aspiration est relié au passage d'adsorption (54), et le dispositif d'adsorption comprend en outre un tube de support (55) et des sections d'extension du passage d'adsorption (54) et du passage de fonctionnement (53 ; 531 ; 532) sont disposées à l'intérieur du tube de support (55).

12. Dispositif d'adsorption selon la revendication 11, dans lequel le dispositif d'adsorption comprend en outre une caractéristique p) supplémentaire, ou les caractéristiques p) et q) :
p) dans lequel une extrémité du tube de support (55) est reliée à la tête d'adsorption (5) et l'autre extrémité du tube de support (55) est pourvue d'une ouverture ;
q) le tube de support (55) a une section de liaison flexible (552).

13. Dispositif d'adsorption selon la revendication 12, dans lequel la section de liaison flexible (552) du tube de support (55) est directement reliée à la tête d'adsorption (5).

14. Dispositif d'adsorption selon la revendication 11, dans lequel le tube de support (55) comprend un premier tube de support (551), une section de liaison flexible (552) et un second tube de support (553) reliés en séquence, et le premier tube de support (551) est relié à la tête d'adsorption (5).
